# EUROPÄISCHE PATENTSCHRIFT

(11) **EP 3 189 752 B1**
(45) Veröffentlichungstag und Bekanntmachung des Hinweises auf die Patenterteilung: **21.08.2019**
(21) Anmeldenummer: 17150699.1
(22) Anmeldetag: 09.01.2017
(51) Int. Cl.: A47F 3/04

(54) **VERFAHREN ZUM REINIGEN EINER LEBENSMITTELKÜHLTHEKE SOWIE LEBENSMITTELKÜHLTHEKE**
METHOD FOR CLEANING A REFRIGERATED FOOD DISPLAY CASE AND REFRIGERATED FOOD DISPLAY CASE
PROCÉDÉ DE NETTOYAGE D'UN COMPTOIR RÉFRIGÉRÉ POUR PRODUITS ALIMENTAIRES ET COMPTOIR RÉFRIGÉRÉ POUR PRODUITS ALIMENTAIRES

(30) Priorität: 08.01.2016 DE 102016100242
(43) Veröffentlichungstag der Anmeldung: 12.07.2017
(73) Patentinhaber: Aichinger GmbH, 90530 Wendelstein (DE)
(72) Erfinder: Kuhlmann, Werner, 90530 Wendelstein (DE)
(74) Vertreter: Reuther, Martin

(56) Entgegenhaltungen:
- DE-A1- 3 313 131
- DE-A1-102013 114 888
- FR-A1- 2 450 429
- US-A1- 2005 138 946
- US-A1- 2014 263 739

## Beschreibung

Die Erfindung betrifft ein Verfahren zum Reinigen einer Lebensmittelkühltheke, bei welchem aus einem Kategorie-5-Reservoir Wasser zu bestimmten Zeiten mittels eines Leitungssystems in einen Warenraum der Lebensmittelkühltheke geleitet wird. Ebenso betrifft die Erfindung eine Lebensmittelkühltheke mit einem Kategorie-5-Reservoir und mit einem Leitungssystem von dem Kategorie-5-Reservoir zu einem Warenraum der Lebensmittelkühltheke.

Es ist mittlerweile hinlänglich bekannt, Lebensmittelkühltheken zu bestimmten Zeiten mit Wasser zu spülen, insbesondere um Staubpartikel oder ähnliches aus dem Warenraum zu entfernen und auch ein regelmäßiges Spülen von Abflüssen der Lebensmittelkühltheke zu gewährleisten, sodass in der Lebensmittelkühltheke und dem zugehörigen Abflusssystem keine stillstehenden Wasser zu finden sind. Entsprechendes offenbart beispielsweise die DE 33 13 131 A1, wobei hier jedoch die Abläufe verhältnismäßig komplex ausgebildet sind. Weitere relevante Dokumente des Standes der Technik sind:
- US 2005/138946 A1,
- DE 10 2013 114888 A1.

Hierbei besteht die Problematik, dass etwaige Rückkontaminationen aus dem Warenraum in die allgemeine Wasserzufuhr unbedingt vermieden werden müssen, sodass das Wasser, welches zu bestimmten Zeiten mittels des Leitungssystems in den Warenraum der Lebensmittelkühltheke geleitet wird, aus einem Kategorie-5-Reservoir bereitgestellt werden muss. Derartige Kategorie-5-Reservoire verhindern wirkungsvoll eine Rückkontamination, in dem die Zufuhr des Wassers in das Kategorie-5-Reservoir von dem Kategorie-5-Reservoir ausreichend getrennt ist.

Es ist Aufgabe vorliegender Erfindung, ein gattungsgemäßes Verfahren zum Reinigen einer Lebensmittelkühltheke sowie eine gattungsgemäße Lebensmittelkühltheke bereitzustellen, die betriebssicher einen erhöhten Reinigungsstandart gewährleisten.

Die Aufgabe der Erfindung wird durch ein Verfahren zum Reinigen einer Lebensmittelkühltheke sowie durch eine Lebensmittelkühltheke mit den Merkmalen der unabhängigen Ansprüche gelöst. Weitere, ggf. auch unabhängig hiervon, vorteilhafte Ausgestaltungen finden sich in den Unteransprüchen sowie der nachfolgenden Beschreibung.

So kann durch ein Verfahren zum Reinigen einer Lebensmittelkühltheke, bei welchem aus einem Kategorie-5-Reservoir Wasser zu bestimmten Zeiten mittels eines Leitungssystems in einen Warenraum der Lebensmittelkühltheke geleitet wird, betriebssicher ein erhöhter Reinigungsstandart gewährleistet werden, wenn durch das Leitungssystem zu bestimmten Zeiten ein Reinigungsmittel geleitet wird.

In Abweichung von bisherigen Verfahren, bei welchem der Warenraum einer Lebensmittelkühltheke zu regelmäßigen Abständen händisch gereinigt wird, gewährleistet die vorliegende Verfahrensweise eine definierte und durch die Festlegung auf bestimmte Zeiten auch zeitlich entsprechend vordefinierte Benetzung des Warenraums bzw. von Teilen des Warenraums einer Lebensmittelkühltheke, was bei einer händischen Reinigung nicht zwingend gegeben ist. Darüber hinaus kann insbesondere auch die Menge an Reinigungsmittel bzw. an gleichzeitig oder im Nachhinein zugeführten Wasser durch die Bestimmung der Zeiten, zu denen Reinigungsmittel bzw. Wasser in den Warenraum geleitet wird, genau definiert werden, was insbesondere auch eine entsprechende Reinigung des Leitungssystems und vor allen Dingen auch von Abwasserleitungen einschließt und was bei händischen Verfahren ohnehin nicht gegeben ist.

Durch die Automatisierungsmöglichkeit besteht darüber hinaus erstmals auch die Chance, Reinigungsmittel einzusetzen, die derart aggressiv sind, dass sie für einen händischen Auftrag bzw. bei der unmittelbaren Anwesenheit von Bedienpersonal nicht bzw. nur unter erschwerten Bedingungen eingesetzt werden können.

Hierbei versteht es sich, dass die bestimmten Zeiten, zu denen Reinigungsmittel durch das Leitungssystem geleitet wird, von den Zeiten, durch denen lediglich Wasser durch das Leitungssystem geleitet wird, abweichen können. Insbesondere ist es, je nach konkreten Erfordernissen auch möglich, das Reinigungsmittel nachts bzw. in Abwesenheit von Personal durch das Leitungssystem zu leiten, um einen unmittelbaren Kontakt zwischen Mensch und dem Reinigungsmittel während dieses Vorgangs ausschließen zu können.

Vorzugsweise wird das Reinigungsmittel nur dann aufgegeben, wenn sich in dem Warenraum keine Waren befinden. Hierdurch kann einfach und wirkungsvoll verhindert werden, dass Reinigungsmittel in der Lebensmittelkühltheke vorgehaltene Waren benetzt und zu einer Gefährdung von Personen, die diese Waren konsumieren, führt. Es versteht sich, dass ggf. auch das Wasser lediglich dann durch das Leitungssystem in den Warenraum der Lebensmittelkühltheke geleitet werden kann, wenn keine Waren in dem Warenraum befindlich sind.

Hierbei kann, je nach konkreter Vorgehensweise, die Anwesenheit von Waren in dem Warenraum maschinell, beispielsweise durch geeignete Sensoren oder sonstige Mittel zur Überprüfung des Warenraums auf Lebensmittel, oder auch manuell, beispielsweise durch einen Druckknopf oder einen Schlüsselschalter oder ähnliche Bestätigungsmittel, durch welche Bedienpersonal bestätigt, dass sich keine Waren in dem Warenraum befinden, sichergestellt werden. Als manuelle Bestätigung kann auch die Beigabe von Reinigungsmittel unmittelbar in das Kategorie-5-Reservoir, beispielsweise von Flüssigreiniger, Tabs oder ähnlichem, gewertet werden.

Insofern kann betriebssicher ein erhöhter Reinigungsstandart bei einer Lebensmittelkühltheke mit einem Kategorie-5-Reservoir und mit einem Leitungssystem von dem Kategorie-5-Reservoir zu einem Warenraum der Lebensmittelkühltheke gewährleistet werden, wenn sich die Lebensmittelkühltheke durch Überprüfungsmittel zur Überprüfung des Warenraums auf Lebensmittel und/oder durch manuelle Betätigungsmittel, dass kein Lebensmittel in dem Warenraum befindlich ist, auszeichnet.

Als Überprüfungsmittel können beispielsweise Ultraschallsensoren oder Infrarotsensoren aber auch Kameras mit einer entsprechenden intelligenten Bildauswertung bzw. Schnüffelsensoren, Lichtschranken oder Wagen, welche beispielsweise die auf einen Warenboden lastenden Gewichte als Maß für die Anwesenheit von Waren nehmen, zur Anwendung kommen.

Manuelle Bestätigungsmittel, durch welche das Personal bestätigen kann, das keine Lebensmittel in dem Warenraum befindlich sind, können - wie vorstehend schon dargelegt - entsprechende Schalter oder Schlüsselschalter sein. Ebenso können Taster, Chipkartenleser oder sonstige ID-Kartenleser genutzt werden, durch welche die Identität der Personen festgehalten werden kann, welche eine entsprechende Bestätigung abgeben. Auch andere Identifikationsmöglichkeiten, die aus dem Stand der Technik bekannt sind, wie beispielsweise Fingerabdruckleser, Augenscanner oder ähnliches können diesbezüglich zur Anwendung kommen. Darüber hinaus versteht es sich, dass als manuelle Bestätigung bereits das Beigeben von Reinigungsmittel in das Kategorie-5-Reservoir oder auch das Öffnen eines Ventils zwischen einem Reinigungsmittelreservoir und dem Kategorie-5-Reservoir oder dem Leitungssystem als eine entsprechende Bestätigung gewertet werden, so dass dann keine Bestätigungsmittel vorgesehen sein müssen, um vom Verfahrensablauf her eine Bestätigung vorliegen zu haben. Hierbei kann beispielsweise auch das Öffnen des Ventils beispielsweise nur dann ermöglicht werden, wenn ein entsprechender Schlüssel steckt, um Fehler oder auch Missbrauch zu vermeiden. Auch sonstige mechanische Sicherungen, mit denen das Aufgeben von Reinigungsmittel ggf. verhindert werden kann, sind denkbar. So kann beispielsweise schon das Vorhandensein eines Warenbodens in einem Warenraum, wenn ein derartiger Warenboden regelmäßig zur Präsentation der Waren in dem Warenraum notwendig oder vorgeschrieben ist, für eine entsprechende Sicherung genutzt werden. So kann beispielsweise die Anwesenheit eines Warenbodens einen entsprechenden Schalter schließen oder öffnen, sodass auf diese Weise gewährleistet ist, dass lediglich bei Abwesenheit des Warenbodens und Vorliegen eines entsprechendes Schaltsignals die Aufgabe von Reinigungsmittel erfolgen kann.

Insbesondere ist es denkbar, dass die Überprüfungsmittel bzw. die manuellen Bestätigungsmittel derart mit dem Leitungssystem bzw. dem Kategorie-5-Reservoir bzw. mit einer entsprechenden Pumpe wechselwirken, dass auch Sicherheitsgründen kein Wasser zu dem Warenraum gelassen wird, wenn nicht betriebssicher die Abwesenheit von Waren festgestellt wird, um Fehlfunktionen der Gestalt zu vermeiden, dass ggf. doch Reinigungsmittel in das Kategorie-5-Reservoir gelangt ist oder das möglicherweise doch ein Ventil geöffnet wurde.

Die vorgenannte Sperre, dass nicht einmal Wasser zu dem Warenraum gelangt, wenn die Überprüfungsmittel bzw. die manuellen Bestätigungsmittel die Abwesenheit von Waren im Warenraum nicht bestätigen, hat zudem den Vorteil, dass auch eine mögliche Kontamination der Lebensmittel durch den das Wasser in den Warenraum bringenden Vorgang, beispielsweise durch Verwirbelungen oder durch ein Aufspritzen oder durch ein Verbreiten von Nebel und ähnlichem, wirkungsvoll vermieden werden können.

Besonders vorteilhaft ist es, wenn die Überprüfungsmittel bzw. die manuellen Bestätigungsmittel mit einem Ventil wechselwirken, welches ein Reinigungsmittelreservoir mit dem Kategorie-5-Reservoir oder dem Leitungssystem verbindet, da dann durch Öffnen und Schließen des Ventils über eine entsprechende Steuerung Fehler sehr einfach auf ein Minimum reduziert werden können. Sollten Reinigungsmitteltabs oder in auflösbaren Hüllen vorportionierte Reinigungsflüssigkeiten bzw. über einen Förderer zuführbares Reinigungspulver oder ähnliches zur Anwendung kommen, so können die Überprüfungsmittel bzw. manuellen Bestätigungsmittel statt mit dem Ventil mit entsprechenden Fördereinrichtungen wirkverbunden sein, sodass lediglich bei der Abwesenheit von Waren in dem Warenraum Reinigungsmittel in das Leitungssystem bzw. in das Kategorie-5-Reservoir gelangen kann.

Zum Leiten des Wassers in den Warenraum kann das Leitungssystem insbesondere Düsen umfassen, durch welche eine gerichtete Verteilung des Wassers gewährleistet werden kann. In der Praxis werden auch schon bei bekannten Lebensmittelkühltheken jeweils zu einem bestimmten Zeitpunkt für eine Lebensmittelkühltheke ungefähr 30 l Wasser in kürzester Zeit in den Warenraum über Düsen geleitet. Geschieht dieses unter entsprechenden Bedingungen mit Reinigungsmittel, so kann durch die Düsen eine entsprechende gezielte Verteilung des Reinigungsmittels gewährleitstet werden.

Vorzugsweise sind die Düsen des Leitungssystems auf wenigstens einen Bereich unterhalb des Warenbodens gerichtet, da dieser einer besonderen Aufmerksamkeit bei der Benetzung mit Wasser zu bestimmten Zeiten bedarf. Oberhalb des Warenbodens, der ggf. ohnehin zu Reinigungszwecken entfernt wird, sind derartige Maßnahmen ggf. nicht notwendig.

Zwar ist es theoretisch denkbar, dass das Wasser bzw. das Reinigungsmittel rein durch die Schwerkraft in den Warenraum gelangt. Vorzugsweise ist hierfür eine Pumpe zwischen dem Kategorie-5-Reservoir und dem Warenraum vorgesehen, was eine besonders effektive Förderung des Wassers bedingt. Baulich wesentlich aufwendiger sind beispielsweise Maßnahmen, mit denen das Kategorie-5-Reservoir unter erhöhten Druck gesetzt wird, um hierdurch das Wasser bzw. das Reinigungsmittel in den Warenraum zu fördern. Es versteht sich, dass ggf. auch eine Pumpe für das Reinigungsmittel vorgesehen sein kann.

Vorzugsweise wird das Reinigungsmittel gemeinsam mit dem Wasser durch das Leitungssystem geleitet. Dieses ermöglicht insbesondere ein Aufgeben des Reinigungsmittels in konzentrierter Form, da dann das Wasser entsprechend verdünnt wird. Auch kann hierdurch sichergestellt wird, dass ausreichend Flüssigkeit vorliegt, um das Reinigungsmittel gezielt in ausreichendem Maße zu verteilen.

Hierbei versteht es ich, dass ggf. Wasser ohne Reinigungsmittel durch das Leitungssystem geleitet werden kann, nachdem Reinigungsmittel durch das Leitungssystem geleitet wurde, um auf diese Weise sicherzustellen, dass etwaige Reinigungsmittelreste aus dem Leitungssystem und auch aus dem Warenraum ausgespült werden, bevor wieder Waren in den Warenraum gelangen.

Gegebenenfalls sind Signaleinrichtungen oder ähnliches vorgesehen, um Bedienpersonal zu warnen, falls ein Reinigungsvorgang und ein hierauf folgendes Spülen lediglich durch Wasser noch nicht ganz abgeschlossen sind.

Beispielsweise kann das Reinigungsmittel dem Wasser bzw. dem Leitungssystem in dem Kategorie-5-Reservoir aufgegeben werden. Dieses ist insbesondere dann von Vorteil, wenn beispielweise Reinigungstabs oder in auflösenden Hüllen befindliches Reinigungsmittel bzw. Reinigungspulver zur Anwendung kommt. Dieses kann dann in den Kategorie-5-Reservoir vermischt bzw. aufgelöst werden, sodass dann Reinigungsmittel in entsprechender Konzentration vorliegt.

Auch ist es möglich, dass Reinigungsmittel dem Leitungssystem über das Kategorie-5-Reservoir aufzugeben, indem Beispielsweise das Reinigungsmittel in das Kategorie-5-Reservoir gegeben wird, wenn sich in diesem kein Wasser befindet. Hierbei ist es insbesondere auch denkbar, dass ein Konzentrat zur Anwendung kommt, welches dann im unteren Bereich des Kategorie-5-Reservoirs konzentriert verbleibt, um dann entweder ohne Wasser in das Leitungssystem gebracht zu werden oder aber gemeinsam mit nachfolgendem Wasser gefördert zu werden. Hier hängt es letztlich von den gewünschten Verfahrensabläufen und auch von den verwendeten Reinigungsmitteln ab, welche Verfahrensweise sich als vorteilhaft erweist.

Es versteht sich, dass das Reinigungsmittel dem Leitungssystem auch nach dem Kategorie-5-Reservoir aufgegeben werden kann. Dieses ist insbesondere dann von Vorteil, wenn ein Reinigungsmittelreservoir über ein Ventil mit dem Leitungssystem verbunden ist.

Es versteht sich, dass die Merkmale der vorstehend bzw. in den Ansprüchen beschriebenen Lösungen gegebenenfalls auch kombiniert werden können, um die Vorteile entsprechend kumuliert umsetzen zu können.

Weitere Vorteile, Ziele und Eigenschaften vorliegender Erfindung werden anhand nachfolgender Beschreibung von Ausführungsbeispielen erläutert, die insbesondere auch in anliegender Zeichnung dargestellt sind. In der Zeichnung zeigen:
- Figur 1: einen schematischen Schnitt durch eine erste Lebensmittelkühltheke; und
- Figur 2: einen schematischen Schnitt durch eine zweite Lebensmittelkühltheke.

Die in den Figuren dargestellten Lebensmittelkühltheken 10 weisen jeweils einen Warenraum 11 auf, in welchem ein Warenboden 12 angeordnet ist, auf welchem Waren dem Publikum bzw. Kunden präsentiert werden können und welcher unter sich einen Bereich 13 aufweist, der letztlich auf einem Bodenkörper 14 angeordnet ist und durch welchen eine über eine Kühlung und einem Ventilator 15 aufrechterhaltende Zirkulation 16 zurückgeführt wird, welche über die Waren oberhalb des Warenbodens 12 streicht.

Es versteht sich, dass ggf. auch andere Arten von Lebensmittelkühltheken zur Umsetzung der Erfindung genutzt werden können, solange bei diesen über ein Kategorie-5-Reservoir 20 Wasser dem Warenraum 11 bzw. einem Bereich 13 des Warenraums zugeführt werden kann.

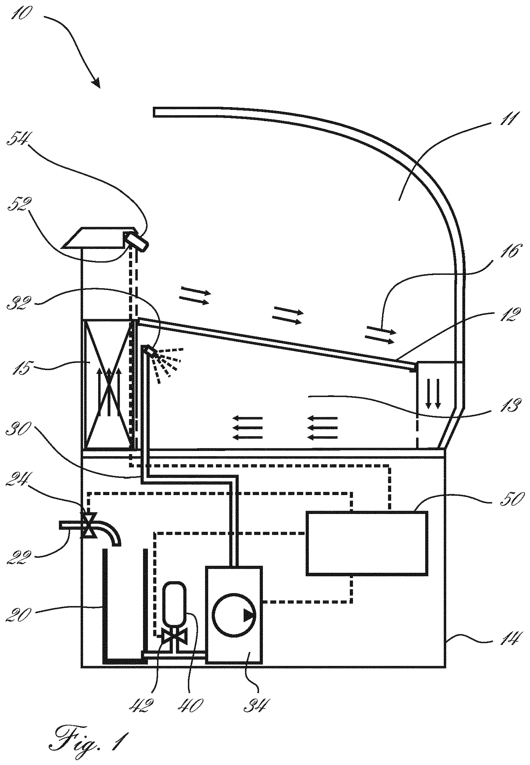

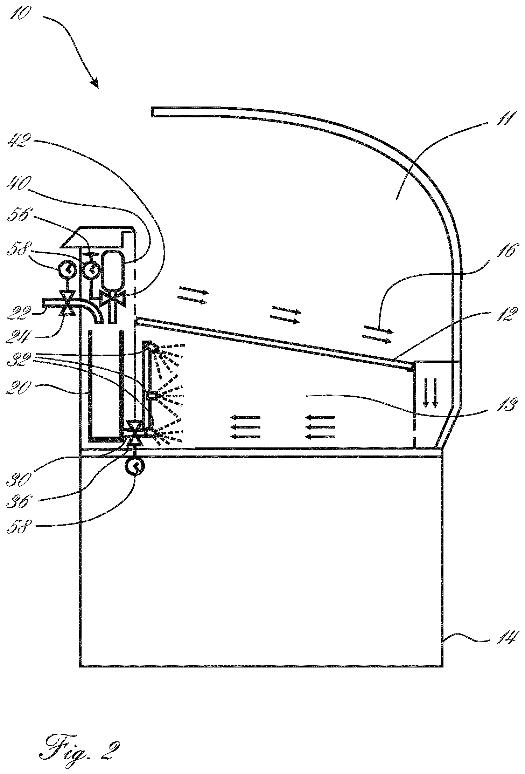

Hierzu weisen die beiden in den Figuren 1 und 2 dargestellten Ausführungsbeispiele zum einen eine Wasserzufuhr 22 auf, über welche mittels eines Ventils 24 Wasser in das Kategorie-5-Reservoir 20 eingefüllt werden kann, wobei zwischen der Wasserzufuhr 22 und dem Kategorie-5-Reservoir 20 sichergestellt ist, das keine Keime oder ähnliches ausgehend von dem Kategorie-5-Reservoir 20 zu der Wasserzufuhr 22 gelangen können.

Darüber hinaus umfassen beide Ausführungsbeispiele ein Leitungssystem 30, welches von dem Kategorie-5-Reservoir zu Düsen 32 reicht, mittels derer das Wasser in den Warenraum 11 bzw. in den Bereich 13 verteilt werden kann.

Bei dem in Figur 1 dargestellten Ausführungsbeispiel ist das Kategorie-5-Reservoir 20 im Bodenkörper 14 angeordnet, wo regelmäßig mehr Platz für entsprechende Einrichtungen verbleibt. Diesen Bodenkörper 14 lässt das Ausführungsbeispiel nach Figur 2 für andere Zwecke bzw. frei und ordnet das Kategorie-5-Reservoir 20 sowie das Leitungssystem 30 im Bereich einer Bedientheke auf Höhe des Bereichs 13 an, wobei in Figur 2 die Kühlung und der Ventilator 15 der Klarheit halber nicht dargestellt sind und letztere sich den Bauraum mit dem Kategorie-5-Reservoir 20 und weiteren zugehörigen Baugruppen teilen.

Bei dem in Figur 1 dargestellten Ausführungsbeispiel wird das Wasser über eine Pumpe 34 zu den Düsen 32 gefördert, wobei auch diese Pumpe im Bodenkörper 14 angeordnet ist.

Darüber hinaus ist bei dem Ausführungsbeispiel in Figur 1 in das Leitungssystem 30 zwischen dem Kategorie-5-Reservoir 20 und der Pumpe 34 ein Reinigungsmittelreservoir 40 angeschlossen, wobei ein Ventil 42 vorgesehen ist, durch welches die Verbindung des Reinigungsmittelreservoirs 40 zu dem Leitungssystem 30 wahlweise geöffnet und geschlossen werden kann.

Die Ventile 24 und 42 sowie die Pumpe 34 können über eine zentrale Steuerung 50 angesteuert werden, welche ihrerseits mit Überprüfungsmittel 52 verbunden ist, die den Warenraum 11 auf die An- bzw. Abwesenheit von Lebensmitteln überprüfen können.

Bei dem vorliegenden Ausführungsbeispiel umfassen die Überprüfungsmittel 52 Sensoren 54, welche als Kamera, Lichtschranken oder Infrarotsensoren ausgebildet sein können. Alternativ hierzu bzw. kumulativ hierzu können auch andere Sensoren, wie beispielsweise Schnüffelsensoren oder ähnliches vorgesehen sein. Es bietet sich auch an, die Anwesenheit des Warenbodens 12 als Überprüfungsmittel 52 im Zusammenspiel mit einem elektrischen Schalter, einer Lichtschranke oder ähnliches zu nutzen, sodass die Steuerung 50 über den elektrischen Schalter bzw. über die Lichtschranke erkennt, ob ein Warenboden 12 eingesetzt ist oder nicht. Letztere Lösung bietet sich insbesondere dann an, wenn betrieblich vorgesehen ist, dass Waren bzw. Lebensmittel ausschließlich auf dem Warenboden 12 präsentiert werden dürfen.

Es versteht sich, dass in abweichenden Ausführungsformen die bei dem Ausführungsbeispiel nach Figur 1 im Bereich des Bodenkörpers 14 vorgesehenen Baugruppen auch an anderer Stelle gemeinsam oder in Teilen vorgesehen sein können. So können diese insbesondere auch auf Höhe der Kühlung und des Ventilators 15 vorgesehen sein. Ebenso können diese Baugruppen ggf. auch separat, beispielsweise mit einem separaten Generatorraum oder ähnlichem angeordnet sein.

Bei dem in Figur 2 dargestellten Ausführungsbeispiel ist auf eine Pumpe 34 verzichtet. Hier schließt sich unmittelbar an das Kategorie-5-Reservoir ein Ventil 36 an, welches das restliche Leitungssystem öffnen und schließen kann, sodass bei geöffnetem Ventil 36 Flüssigkeit aus dem Kategorie-5-Reservoir 20 zu den Düsen 32 gelangen und von dort in den Warenraum 11 verteilt werden kann. Es versteht sich hierbei, dass die oberen Düsen 32 nur solange mit Flüssigkeit versorgt werden, solange der Wasserspiegel hier ausreichend ist. Dementsprechend kann es auch vorteilhaft sein, bei einer derartigen Ausgestaltung das Kategorie-5-Reservoir 20 höher, ggf. auch an separater Stelle, sehr hoch anzuordnen.

Um Reinigungsmittel aufgeben zu können, weist auch die Anordnung nach Figur 2 ein Reinigungsmittelreservoir auf, welches über ein Ventil 42 angesteuert Reinigungsmittel in das Kategorie-5-Reservoir 20 geben kann.

Damit letzteres nicht erfolgt, ist das Ventil 42 des Ausführungsbeispiels nach Figur 2 mit Bestätigungsmitteln 56 verbunden, die bei diesem Ausführungsbeispiel als Taster ausgebildet sind. Hierdurch kann das Personal bestätigen, dass keine Waren in dem Warenraum 11 befindlich sind. Gegebenenfalls können diese Bestätigungsmittel 56 bzw. ein derartiger Taster auch mit dem Warenboden wechselwirken, sodass diese letztlich als Überprüfungsmittel 52, wie sie bereits anhand des Ausführungsbeispiels nach Figur 1 geschrieben sind, wirken. Es versteht sich, dass hier alternativ auch andere Bestätigungsmittel, wie beispielsweise Fingerabdrucksensoren, Chipkarten oder ähnliches, vorgesehen sein können. Ebenso können derartige Bestätigungsmittel auch bei dem Ausführungsbeispiel nach Figur 1 kumulativ bzw. alternativ zu den Überprüfungsmitteln 52 vorgesehen sein.

Statt einer zentralen Steuerung weist das Ausführungsbeispiel nach Figur 2 für jedes Ventil 42, 36, 24 eine Zeitschaltuhr 58 auf, sodass auf baulich sehr einfache Weise ein Entleeren des Kategorie-5-Reservoirs 20, ob mit oder ohne Reinigungsmittel, einerseits und die Zugabe von Reinigungsmittel aus dem Reinigungsmittelreservoire 40 sowie die Zugabe von Wasser über die Wasserzufuhr 22 jeweils in das Kategorie-5-Reservoir 20 andererseits einfach und betriebssicher hinsichtlich der Zeiten definiert werden kann. Es versteht sich, dass in abweichenden Ausführungsformen ggf. auch eine zentrale Zeitschaltuhr oder Steuerung mit einer entsprechenden Anzahl an Kanälen zur Anwendung kommen kann.

Darüber hinaus kann, wenn eine manuelle Aufgabe von Reinigungsmittel vorgesehen sein soll, auf das Reinigungsmittelreservoir 40 mit dem Ventil 42 und den Bestätigungsmittel 56 verzichtet werden, wenn stattdessen lediglich Reinigungsmittel zu bestimmten Zeiten dem Kategorie-5-Reservoir aufgegeben wird. Es versteht sich, dass letzteres ggf. auch bei dem Ausführungsbeispiel nach Figur 1 realisiert werden kann, wenn auch dort auf das Reinigungsmittelreservoir 40 und das Ventil 42 verzichtet werden soll. Ebenso ist es denkbar, dass sich das Reinigungsmittelreservoir 40 bei dem Ausführungsbeispiel nach Figur 1 in das Kategorie-5-Reservoir 20 öffnet, wie dieses bei dem Ausführungsbeispiel nach Figur 2 der Fall ist.

Es versteht sich, dass die vorstehend beschriebene Aufgabe von Reinigungsmittel auch bei anders ausgestalteten Lebensmittelkühltheken vorteilhaft zum Einsatz kommen kann, solange diese Wasser über einen Kategorie-5-Reservoir und ein Leitungssystem zu einem Warenraum fördern.

**Bezugszeichenliste:**

| | | | |
|---|---|---|---|
| 10 | Lebensmittelkühltheke | 32 | Düse |
| 11 | Warenraum | 34 | Pumpe |
| 12 | Warenboden | 36 | Ventil |
| 13 | Bereich | | |
| 14 | Bodenkörper | 40 | Reinigungsmittelreservoir |
| 15 | Kühlung und Ventilator | 42 | Ventil |
| 16 | Zirkulation | | |
| | | 50 | Steuerung |
| 20 | Kategorie-5-Reservoir | 52 | Überprüfungsmittel |
| 22 | Wasserzufuhr | 54 | Sensor |
| 24 | Ventil | 56 | Bestätigungsmittel |
| | | 58 | Zeitschaltuhr |
| 30 | Leitungssystem | | |

## Patentansprüche

1. Verfahren zum Reinigen einer Lebensmittelkühltheke (10), wobei aus einem Kategorie-5-Reservoir (20) Wasser zu bestimmten Zeiten mittels eines Leitungssystems (30) in einen Warenraum (11) der Lebensmittelkühltheke (10) geleitet wird und durch das Leitungssystem (30) zu bestimmten Zeiten ein Reinigungsmittel geleitet wird, **dadurch gekennzeichnet, dass** die Zufuhr des Wassers in das Kategorie-5-Reservoir zur Verhinderung einer Rückkontamination von dem Kategorie-5-Reservoir getrennt ist.

2. Reinigungsverfahren nach Anspruch 1, **dadurch gekennzeichnet, dass** das Reinigungsmittel nur dann aufgegeben wird, wenn sich in dem Warenraum (11) keine Waren befinden.

3. Reinigungsverfahren nach Anspruch 1 oder 2, **dadurch gekennzeichnet, dass** das Reinigungsmittel gemeinsam mit dem Wasser durch das Leitungssystem (30) gleitet wird.

4. Reinigungsverfahren nach einem der Ansprüche 1 bis 3, **dadurch gekennzeichnet, dass** das Reinigungsmittel dem Wasser und/oder dem Leitungssystem (30) in dem Kategorie-5-Reservoir (20) und/oder dem Leitungssystem (30) über das Kategorie-5-Reservoir (20) und/oder dass das Reinigungsmittel dem Leitungssystem (30) nach dem Kategorie-5-Reservoir (20) aufgegeben wird.

5. Reinigungsverfahren nach einem der Ansprüche 1 bis 4, **dadurch gekennzeichnet, dass**, nachdem Reinigungsmittel durch das Leitungssystem (30) geleitet wurde, Wasser ohne Reinigungsmittel durch das Leitungssystem (30) geleitet wird.

6. Lebensmittelkühltheke (10) aufweisend ein Kategorie-5-Reservoir (20), ein Leitungssystem (30) von dem Kategorie-5-Reservoir (20) zu einem Warenraum (11) der Lebensmittelkühltheke (10) sowie ein Reinigungsmittelreservoir (40), welches mit dem Kategorie-5-Reservoir (20) oder dem Leitungssystem (30) über ein Ventil (42) verbunden ist, **dadurch gekennzeichnet, dass** die Zufuhr von Wasser in das Kategorie-5-Reservoir zur Verhinderung einer Rückkontamination von dem Kategorie-5-Reservoir getrennt ist.

7. Lebensmittelkühltheke (10) aufweisend ein Kategorie-5-Reservoir (20), ein Leitungssystem (30) von dem Kategorie-5-Reservoir (20) zu einem Warenraum (11) der Lebensmittelkühltheke (10) sowie Mittel (52) zur Überprüfung des Warenraums (11) auf Lebensmittel und/oder manuelle Bestätigungsmittel (56), dass keine Lebensmittel in dem Warenraum (11) befindlich sind, **dadurch gekennzeichnet, dass** die Zufuhr von Wasser in das Kategorie-5-Reservoir zur Verhinderung einer Rückkontamination von dem Kategorie-5-Reservoir getrennt ist.

8. Lebensmittelkühltheke nach Anspruch 6 oder 7, **dadurch gekennzeichnet, dass** der Warenraum (11) einen Warenboden (12) umfasst und Düsen (32) des Leitungssystems auf wenigstens einen Bereich (13) unterhalb des Warenbodens (12) gerichtet sind.

9. Lebensmittelkühltheke nach einem der Ansprüche 6 bis 8, **gekennzeichnet durch** eine Pumpe (34) zwischen dem Kategorie-5-Reservoir (20) und dem Warenraum (11).

10. Reinigungsverfahren nach einem der Ansprüche 1 bis 5 oder Lebensmittelkühltheke nach einem der Ansprüche 6 bis 9, **gekennzeichnet durch** eine Wasserzufuhr (22), über welche mittels eines Ventils (24) Wasser in das Kategorie-5-Reservoir (20) eingefüllt werden kann, wobei zwischen der Wasserzufuhr (22) und dem Kategorie-5-Reservoir (20) sichergestellt ist, dass keine Keime oder ähnliches ausgehend von dem Kategorie-5-Reservoir (20) zu der Wasserzufuhr (22) gelangen können.

## Claims

1. A method for cleaning a refrigerated food counter (10), wherein water from a category 5 reservoir (20) is at specific times guided by means of a piping system (30) into a product space (11) of the refrigerated food counter (10), and a cleaning agent is at specific times guided through the piping system (30), **characterized in that** the supply of water into the category 5 reservoir for preventing reverse contamination is separated from the category 5 reservoir.

2. The cleaning method according to claim 1, **characterized in that** the cleaning agent is only dispensed when no product is present in the product space (11).

3. The cleaning method according to claim 1 or 2, **characterized in that** the cleaning agent is guided through the piping system (30) together with the water.

4. The cleaning method according to one of claims 1 to 3, **characterized in that** the cleaning agent is dispensed into the water and/or the piping system (30) in the category 5 reservoir (20) and/or the piping system (30) via the category 5 reservoir (20) and/or that the cleaning agent is dispensed into the piping system (30) after the category 5 reservoir (20).

5. The cleaning method according to one of claims 1 to 4, **characterized in that**, after cleaning agent has been guided through the piping system (30), water without cleaning agent is guided through the piping system (30) .

6. A refrigerated food counter (10) having a category 5 reservoir (20), a piping system (30) from the category 5 reservoir (20) to a product space (11) of the refrigerated food counter (10) as well as a cleaning agent reservoir (40) that is connected with the category 5 reservoir (20) or the piping system (30) by way of a valve (42), **characterized in that** the supply of water into the category 5 reservoir for preventing reverse contamination is separated from the category 5 reservoir.

7. A refrigerated food counter (10) having a category 5 reservoir (20), a piping system (30) from the category 5 reservoir (20) to a product space (11) of the refrigerated food counter (10) as well as means (52) for checking the product space (11) for food and/or manual activation means (56) to verify that no food is in the product space (11), **characterized in that** the supply of water into the category 5 reservoir for preventing reverse contamination is separated from the category 5 reservoir.

8. The refrigerated food counter according to claim 6 or 7, **characterized in that** the product space (11) comprises a product floor (12) and nozzles (32) of the piping system are directed at least at an area (13) under the product floor (12).

9. The refrigerated food counter (10) according to one of claims 6 to 8, **characterized by** a pump (34) between the category 5 reservoir (20) and the product space (11).

10. The cleaning method according to one of claims 1 to 5 or refrigerated food counter according to one of claims 6 to 9, **characterized by** a water supply (22) which can supply water into the category 5 reservoir (20) by way of a nozzle (24), wherein it is ensured between the water supply (22) and the category 5 reservoir (20) that no germs of the like can get from the category 5 reservoir (20) to the water supply (22).

## Revendications

1. Procédé pour le nettoyage d'un comptoir réfrigéré pour produits alimentaires (10), dans lequel de l'eau est guidée à partir d'un réservoir catégorie 5 (20) à des moments précis vers un compartiment à produits (11) du comptoir réfrigéré pour produits alimentaires (10) au moyen d'un système de conduites (30) et un agent de nettoyage est guidé à des moments précis à travers le système de conduites (30),
**caractérisé en ce que**
l'alimentation de l'eau dans le réservoir catégorie 5 est séparée du réservoir catégorie 5 pour éviter une contamination inverse.

2. Procédé de nettoyage selon la revendication 1,
**caractérisé en ce que** l'agent de nettoyage n'est ajouté que lorsqu'aucun produit ne se trouve dans le compartiment à produits (11).

3. Procédé de nettoyage selon la revendication 1 ou 2,
**caractérisé en ce que** l'agent de nettoyage est guidé à travers le système de conduites (30) ensemble avec l'eau.

4. Procédé de nettoyage selon l'une des revendications 1 à 3, **caractérisé en ce que** l'agent de nettoyage est ajouté à l'eau et/ou au système de conduites (30) dans le réservoir catégorie 5 (20) et/ou au système de conduites (30) par le biais du réservoir catégorie 5 (20) et/ou **en ce que** l'agent de nettoyage est ajouté au système de conduites (30) après le réservoir catégorie 5 (20).

5. Procédé de nettoyage selon l'une des revendications 1 à 4, **caractérisé en ce qu'**une fois que l'agent de nettoyage a été guidé à travers le système de conduites (30), de l'eau sans agent de nettoyage est guidée à travers le système de conduites (30).

6. Comptoir réfrigéré pour produits alimentaires (10) comprenant un réservoir catégorie 5 (20), un système de conduites (30) allant du réservoir catégorie 5 (20) à un compartiment à produits (11) du comptoir réfrigéré pour produits alimentaires (10) ainsi qu'un réservoir d'agent de nettoyage (40), lequel est relié au réservoir catégorie 5 (20) ou au système de conduites (30) par le biais d'une vanne (42),
**caractérisé en ce que**
l'alimentation d'eau dans le réservoir catégorie 5 est séparée du réservoir catégorie 5 pour éviter une contamination inverse.

7. Comptoir réfrigéré pour produits alimentaires (10) comprenant un réservoir catégorie 5 (20), un système de conduites (30) allant du réservoir catégorie 5 (20) à un compartiment à produits (11) du comptoir réfrigéré pour produits alimentaires (10), ainsi qu'un moyen (52) permettant de détecter la présence de produits alimentaires dans le compartiment à produits (11) et/ou des moyens manuels (56) permettant de confirmer l'absence de produits alimentaires dans le compartiment à produits (11),
**caractérisé en ce que**
l'alimentation d'eau dans le réservoir catégorie 5 est séparée du réservoir catégorie 5 pour éviter une contamination inverse.

8. Comptoir réfrigéré pour produits alimentaires selon la revendication 6 ou 7, **caractérisé en ce que** le compartiment à produits (11) comporte un plancher à produits (12) et des buses (32) du système de conduites sont orientées au moins vers une région (13) située en dessous du plancher à produits (12).

9. Comptoir réfrigéré pour produits alimentaires selon l'une des revendications 6 à 8, **caractérisé par** une pompe (34) entre le réservoir catégorie 5 (20) et le compartiment à produits (11).

10. Procédé de nettoyage selon l'une des revendications 1 à 5 ou comptoir réfrigéré pour produits alimentaires selon l'une des revendications 6 à 9, **caractérisé par** une arrivée d'eau (22) par le biais de laquelle de l'eau peut être introduite dans le réservoir catégorie 5 (20) à l'aide d'une vanne (24), dans lequel, entre l'arrivée d'eau (22) et le réservoir catégorie 5 (20), il est assuré qu'aucun germe ou similaire ne peut passer du réservoir catégorie 5 (20) à l'arrivée d'eau (22).
